# DEMANDE DE BREVET EUROPEEN

(11) **EP 0 612 499 A1**
(43) Date de publication de la demande: **31.08.1994**
(21) Numéro de dépôt: 94400021.5
(22) Date de dépôt: 05.01.1994
(51) Int. Cl.: A61B 5/117, A43D 1/02

(54) **Appareil permettant de réaliser des empreintes par impression à l'encre**

(30) Priorité: 29.01.1993 FR 9300943
(71) Demandeur: ETABLISSEMENTS COBLENTZ Société Anonyme, F-75010 Paris (FR)
(72) Inventeur: Coblentz, Serge Bernard, F-75010 Paris (FR)
(74) Mandataire: Lefebure, Gérard

(57) **Abrégé**

Dans cet appareil comprenant un boîtier (2), un support (7) ayant une surface d'appui pour supporter au moins une feuille de papier (8) destinée à recevoir une empreinte, une membrane (4) en une matière élastomère tendue dans le boîtier et ayant une face exposée à l'extérieur du boîtier, et un rouleau encreur (6) fixé a une tirette (7) mobile dans le boîtier pour encrer l'autre face de la membrane, et comportant un noyau cylindrique dur (6a) pourvu d'une garniture extérieure (6b) imprégnée d'encre, la garniture (6b) est constituée par un bandage cylindrique épais en une matière plastique spongieuse microporeuse contenant dans sa masse une encre à base de solvants lourds, non volatils, hygroscopiques, non plastifiants pour ladite matière plastique et capables d'exsuder lentement vers l'extérieur du bandage pour assurer l'encrage.

## Description

La présente invention concerne un appareil permettant de réaliser des empreintes par impression à l'encre, du type comprenant un boîtier, un support ayant une surface d'appui pour supporter au moins une feuille de papier destinée à recevoir une empreinte, une membrane en une matière élastomère, qui est tendue dans le boîtier et possède une première face orientée vers ladite feuille de papier et une seconde face dont une grande partie au moins est exposée à l'extérieur du boîtier, et un rouleau encreur qui est en contact avec la première face de la membrane et fixé à une tirette montée mobile dans le boîtier pour déplacer le rouleau encreur le long de la première face de la membrane afin d'encrer celle-ci, ledit rouleau encreur comportant un noyau cylindrique dur pourvu d'une garniture extérieure imprégnée d'encre.

Les appareils de ce type sont utilisables notamment, mais non exclusivement, par les podologues et les orthopédistes pour obtenir par exemple des empreintes de la voûte plantaire de leurs patients.

Dans les appareils connus du type sus-indiqué, le noyau cylindrique dur du rouleau encreur est habituellement constitué par un corps cylindrique plein en bois ou en une matière plastique dure, et la garniture extérieure du rouleau encreur est habituellement constituée par une gaine d'étoffe (feutre, velours, etc...) d'épaisseur relativement faible et pouvant être imprégnée par des encres d'imprimerie ou autres. Le rouleau encreur est fixé à la tirette, qui permet d'en assurer le déplacement, par des vis ou des rivets situés aux extrémités du rouleau et tenant lieu d'axe de rotation pour celui-ci.

Ces appareils connus nécessitent une intervention manuelle pour imprégner d'encre le rouleau, dès la première utilisation de l'appareil, et la réimprégnation d'encre à chaque fois que cela est rendu nécessaire par l'élimination progressive de l'encre qui est extraite de la gaine en tissu à chaque enduction de la première surface de la membrane. Cette imprégnation est usuellement faite manuellement par badigeonnage du rouleau à l'aide d'un distributeur, d'une bouteille ou autre flacon délivrant une certaine dose d'encre ou produit similaire.

Une telle opération est très délicate et source de salissures peu souhaitables dans un cabinet de soins. En outre, la répartition de l'encre sur la gaine en tissu du rouleau encreur ne peut être uniforme et elle est difficilement maîtrisée en quantité. Pour obtenir une répartition à peu près convenable de l'encre sur la gaine en tissu et par suite aussi sur la première face de la membrane, il faut faire faire au rouleau plusieurs allers et retours sur la première face de la membrane au moyen de la tirette, et il faut effectuer plusieurs essais d'impression d'empreintes avant de parvenir à une qualité d'impression acceptable. Tout ceci représente une perte de temps et un gâchis d'encre et de papier.

En outre, dans les appareils connus, le remplacement du rouleau (gaine de tissu encreur usée ou endommagée par l'usage de l'appareil) est conditionné par le type de fixation du rouleau à la tirette :
1°) Dans le cas d'un rouleau fixé de façon démontable à la tirette par des vis, on rencontre les problèmes suivants :
   a -difficulté d'accès aux têtes de vis;
   b -nécessité de posséder un outil approprié (tournevis) répondant à la forme et aux dimensions de la tête de vis;
   c -difficulté plus ou moins grande d'extraire les vis, selon la dureté du bois ou du matériau constituant le noyau ou corps du rouleau, ou encore à cause d'une éventuelle oxydation des vis dans le corps du rouleau.
2°) Dans le cas où le rouleau est fixé de façon non démontable à la tirette par des rivets ou par des axes battus ou matricés, on doit alors remplacer l'ensemble rouleau/tirette alors que seul l'état du rouleau nécessiterait son remplacement.

La présente invention a donc pour but de fournir un appareil du type sus-indiqué, permettant d'obtenir des empreintes par impression à l'encre, qui soit d'une utilisation nettement plus simple, plus efficace et plus propre pour l'utilisateur de l'appareil.

En particulier, la présente invention a pour but de fournir un appareil du type sus-indiqué, dont le rouleau encreur n'a pas besoin d'être imprégné ou réimprégné d'encre par l'utilisateur.

L'invention a également pour but de fournir un appareil du type sus-indiqué, dans lequel le rouleau peut être facilement et rapidement remplacé par un rouleau neuf lorsque l'encre est épuisée.

A cet effet, l'appareil selon la présente invention est caractérisé en ce que la garniture extérieure du rouleau encreur est constituée par un bandage cylindrique épais en une matière plastique spongieuse microporeuse contenant dans sa masse une encre à base de solvants lourds, non volatils, hygroscopiques, non plastifiants pour ladite matière plastique et capables d'exsuder lentement vers l'extérieur du bandage pour assurer l'encrage.

D'autres caractéristiques et avantages de la présente invention ressortiront mieux au cours de la description qui va suivre d'une forme de réalisation de l'invention, donnée à titre d'exemple en référence aux dessins annexés sur lesquels :
- la figure 1 est une vue en coupe longitudinale d'un appareil selon la présente invention, la coupe étant faite suivant la ligne I-I de la figure 2;
- la figure 2 est une demie-vue en coupe transversale de l'appareil suivant la ligne II-II de la figure 1.

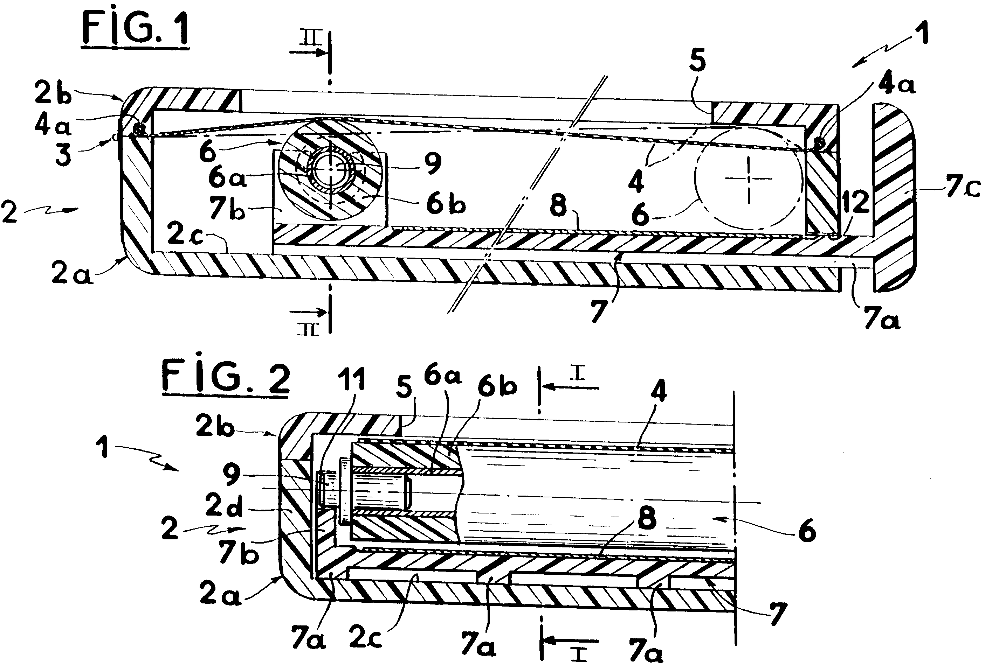

L'appareil 1 représenté dans les figures 1 et 2 comprend un boîtier 2, qui a par exemple une forme parallélépipédique et qui est de préférence réalisé en deux parties, à savoir une partie inférieure 2a relativement profonde et une partie supérieure ou couvercle 2b, qui peut être séparée ou écartée de la partie inférieure 2a pour donner accès à l'intérieur du boîtier. Dans la forme de réalisation représentée sur le dessin, le couvercle 2b est relié à la partie inférieure 2a du boîtier par une articulation 3. Cette articulation 3 peut être par exemple constituée par une charnière de type classique ou, dans le cas où le boîtier 2 est en matière plastique, la charnière peut être réalisée de façon connue sous forme d'un mince voile en une matière plastique souple et capable de résister à des flexions répétées, formé d'un seul tenant, par moulage, avec les parties 2a et 2b du boîtier 2. Dans d'autres formes de réalisation, le couvercle 2b et la partie inférieure 2a pourraient être assemblés l'un à l'autre par un joint du type à emboîtement.

Comme montré sur le dessin, l'appareil 1 comprend en outre une membrane 4 en une matière élastomère facilement déformable, par exemple en latex, qui est tendue à l'intérieur du boîtier 2, à distance du fond 2c de celui-ci. Par exemple, la membrane 4 comporte une partie plus épaisse 4a, par exemple en forme de bourrelets, le long de ses côtés opposés, ces bourrelets étant emboîtés à force dans des rainures de forme correspondante prévues dans au moins deux bords opposés correspondants du couvercle 2b. Bien entendu, la membrane 4 pourrait être fixée au boîtier 2 par d'autres moyens, par exemple par collage ou par pincement de son bord périphérique au moyen de barrettes de fixation ou d'un cadre de fixation pouvant être fixé de manière détachable au bord du couvercle 2b ou au bord de la partie inférieure 2a du boîtier. Comme cela est bien visible dans les figures 1 et 2, le couvercle 2b présente une large ouverture 5, par laquelle la plus grande partie de la face supérieure de la membrane 4 est exposée à l'extérieur du boîtier. Ce dernier peut comporter des moyens de verrouillage (non montrés) de n'importe quel type connu pour maintenir le couvercle fermé, et permettant de l'ouvrir quand on le désire.

L'appareil 1 comprend en outre un rouleau encreur 6 monté sur une tirette 7 qui peut être déplacée à l'intérieur du boîtier 2, sous la membrane 4, pour encrer cette dernière. Comme montré, la tirette 7 peut être réalisée sous la forme d'une planchette qui présente, dans sa face supérieure, un évidemment peu profond apte à recevoir au moins une feuille de papier 8 sur laquelle une empreinte désirée peut être imprimée au moyen de la membrane 4 utilisée comme tampon d'impression. Sur sa face inférieure, la tirette ou planchette 7 comporte un certain nombre de nervures longitudinales 7a qui facilitent le glissement de la tirette sur le fond 2c du boîtier 2, en réduisant les frottements entre ces deux éléments, et qui servent aussi à renforcer et à rigidifier la tirette pour lui permettre de résister à la pression d'impression appliquée à la feuille de papier 8 à travers la membrane 4.

A une de ses extrémités, la tirette ou plaquette 7 comporte deux flasques espacés 7b (un seul de ces deux flasques est visible dans les figures 1 et 2), qui sont d'un seul tenant avec la tirette et servent de paliers pour le rouleau encreur 6 comme on le verra plus loin.

Le rouleau encreur 6 comporte un noyau cylindrique dur 6a, qui est garni extérieurement d'un bandage cylindrique épais 6b en une matière plastique spongieuse microporeuse contenant dans sa masse une encre à base de solvants lourds, non volatils, hygroscopiques, non plastifiants pour la matière plastique constituant le bandage 6a et capables d'exsuder lentement vers l'extérieur du bandage pour assurer l'encrage de la membrane 4.

A titre de solvants lourds pour la préparation de l'encre imprégnant le bandage 6b, on peut utiliser notamment, mais non exclusivement, des dialcools ou des trialcools additionnés de colorants appropriés à la couleur désirée. A titre de matière plastique spongieuse pour la fabrication du bandage 6b, on peut utiliser par exemple une émulsion de chlorure de vinyle additionnée d'une quantité appropriée d'agents plastifiants pour obtenir la souplesse désirée dudit bandage. A cet égard, on notera que, lorsque le rouleau encreur est en service, c'est-à-dire quand il est imprégné d'encre, sa surface extérieure ne doit pas être altérable par pression, c'est-à-dire ne doit pas conserver de déformation permanente par pression, mais doit rester aussi uniforme que possbile pour garantir pendant toute sa durée de vie un encrage uniforme de la membrane 4. Ceci étant précisé, le bandage 6b peut être réalisé par mélange de l'émulsion de chlorure de vinyle et de l'encre préparées au préalable, par malaxage de ce mélange jusqu'à l'obtention d'une pâte homogène, puis par moulage et cuisson de la pâte dans un moule approprié pour obtenir un bandage ayant le diamètre extérieur désiré. Au besoin, on peut se servir du noyau 6a comme partie du moule, de telle façon que le bandage 6b soit directement formé sur le noyau 6a.

A chacune de ses extrémités, le noyau 6a du rouleau encreur 6 est pourvu d'un tourillon 9. Dans le cas où le noyau 6a est constitué par un tube en métal, par exemple en acier, ou en une matière plastique dure, chaque tourillon 9 peut être lui-méme realisé sous la forme d'un insert, métallique ou en matière plastique, emboité à force dans le tube formant le noyau 6a, comme montré dans les figures et 2. A titre de variante, si le noyau 6a est réalisé sous la forme d'un noyau plein en métal ou en matière plastique dure, chaque tourillon 9 peut être formé d'une seule pièce avec le noyau 6a.

Chaque flasque 7b de la tirette 7 peut avantageusement comporter dans son bord supérieur une encoche 11 ayant un profil en partie circulaire, dont le rayon correspond au diamètre des tourillons 9 et qui s'étend sur un arc d'un peu plus de 180°. De cette manière, chaque tourillon 9 peut être introduit à force dans l'encoche 11 du flasque 7b correspondant et maintenu dans cette encoche par encliquetage élastique. Ainsi, le rouleau encreur 6 peut être facilement et rapidement fixé à la tirette 7 ou dégagé de celle-ci par exemple pour être remplacé par un rouleau neuf. A cet égard, on notera que pour une fréquence d'utilisation moyenne de l'appareil, c'est-à-dire environ huit à dix utilisations par semaine, la durée de vie du rouleau encreur 6 de l'appareil selon l'invention est d'au moins douze mois. A titre de comparaison, pour une même fréquence d'utilisation, le rouleau encreur avec garniture en tissu (feutre ou velours) utilisé dans les appareils antérieurement connus nécessitait un réencrage une fois toutes les quatre à cinq semaines, et cela même en l'absence totale d'utilisation durant cette période.

A titre indicatif, le noyau 6a du rouleau encreur 6 peut avoir par exemple un diamètre intérieur de 8mm et un diamètre extérieur de 10mm, et le bandage 6b peut avoir un diamètre extérieur de 22,5mm.

La tirette 7 est en contact glissant avec le fond 2c du boîtier 2, comme on l'a déjà indiqué plus haut, et elle passe à travers une fente 12 prévue dans l'une des parois latérales du boîtier, près du fond de celui-ci, comme on peut le voir dans la partie droite de la figure 1. Ainsi, la tirette 7 est guidée par la fente 12, par le fond 2c du boîtier et par les deux parois latérales du boîtier 2 qui s'étendent le long des côtés longitudinaux de la tirette 7, comme la paroi 2d de la figure 2. Bien que cela ne soit pas montré dans la figure 2, la nervure 7a qui longe le bord longitudinal de la tirette 7 adjacent à la paroi 2d ainsi que la nervure qui longe le bord longitudinal opposé de la tirette 7 peuvent être élargies au moins au droit des deux flasques 7b de façon à faire saillie sur les côtés de la tirette 7 et à être en contact glissant avec les parois latérales adjacentes du boîtier, comme la paroi 2d de la figure 2, afin d'améliorer le guidage latéral de la tirette 7.

Comme cela est plus particulièrement visible dans la figure 1, la partie 7c de la tirette 7 qui se trouve à l'extérieur du boîtier 2 peut être avantageusement réalisée sous la forme d'une poignée pour faciliter la manoeuvre de la tirette.

Comme cela est également visible dans la figure 1, les dimensions des deux flasques 7b de la tirette 7, en particulier leur hauteur, et le diamètre extérieur du bandage cylindrique 6b sont choisis de telle façon que la partie périphérique dudit bandage qui est en contact avec la membrane élastique 4 soit à une distance du fond 2c du boîtier 2 sensiblement plus grande que la distance à laquelle les côtés de la membrane 4 sont maintenus par rapport au fond 2c du boîtier.

Comme la membrane 4 est tendue élastiquement entre ses deux côtés opposés par lesquels elle est fixée au boîtier, il en résulte que la membrane 4 est fermement appliquée élastiquement contre la partie supérieure du bandage 6b du rouleau encreur 6. Ceci contribue avec le type particulier de rouleau encreur utilisé dans l'appareil de la présente invention à l'obtention d'un encrage uniforme de la face inférieure de la membrane 4 lorsque le rouleau encreur 6 est déplacé le long de celle-ci par la tirette 7.

L'appareil 1 décrit ci-dessus fonctionne de la manière suivante. Avant chaque utilisation de l'appareil, c'est-à-dire avant la production d'une empreinte, la tirette 7 est déplacée depuis sa position complètement rentrée dans le boîtier 2 juqu'à sa position d'extraction maximale à l'extérieur du boîtier 2 (dans cette dernière position, le rouleau encreur 6 occupe la position montrée en traits mixtes dans la figure 1). Dans la position d'extraction maximale de la tirette 7, une feuille de papier 8 est ensuite placée dans l'évidement peu profond sur la face supérieure de la tirette 7, s'il n'y avait pas déjà une feuille de papier, puis la tirette 7 est à nouveau rentrée à fond dans le boîtier 2. Ce mouvement d'aller et retour de la tirette 7 a pour effet de provoquer l'encrage de la face inférieure de la membrane 4 par le rouleau encreur 6. L'appareil 1 est alors près à être utilisé pour imprimer une empreinte, par exemple l'empreinte d'une voûte plantaire, sur la feuille 8. Il suffit pour cela d'introduire le pied dénudé à travers l'ouverture 5 du couvercle 2b et d'exercer avec le pied une pression sur la membrane 4 pour la déformer élastiquement jusqu'à ce qu'elle vienne en contact avec la feuille 8. Après avoir maintenu le contact pendant quelques secondes, le pied peut être retiré de l'appareil. La feuille de papier 8 ainsi imprimée peut être ensuite extraite de l'appareil de deux manières. Si l'appareil n'a pas besoin d'être réutilisé immédiatement après l'impression qui vient d'être réalisée, la feuille de papier 8 peut alors être extraite du boîtier 2 en ouvrant le couvercle 2b. Celui-ci peut être ensuite refermé après avoir éventuellement placé une feuille vierge sur la tirette 7. Par contre, si l'appareil doit à nouveau être utilisé pour imprimer une seconde empreinte, la tirette 7 est extraite du boîtier 2, la feuille imprimée 8 est enlevée de la tirette et une feuille vierge est mise à sa place sur la tirette 7 qui est ensuite rentrée à fond dans le boîtier 2. L'appareil 1 est alors prêt pour réaliser la seconde impression.

Il va de soi que la forme d'exécution de l'invention qui a été décrite ci-dessus a été donnée à titre d'exemple purement indicatif et nullement limitatif, et que de nombreuses modifications peuvent être facilement apportées par l'homme de l'art sans pour autant sortir du cadre de l'invention. C'est ainsi notamment qu'un élément amovible de protection, comme par exemple une trappe mobile ou un couvercle additionnel, peut être prévu pour obturer l'ouverture 5 en cas d'inutilisation prolongée de l'appareil, afin de protéger la membrane 4 contre les chocs et éviter l'accumulation de poussière sur ladite membrane.

Par ailleurs, au lieu d'être fixée au couvercle 2b, la membrane 4 peut être fixée de manière détachable à la partie inférieure 2a du boîtier. Dans ce cas, le couvercle 2b peut être dépourvu d'ouverture 5 et il a alors simplement un rôle de protection de la membrane pendant les périodes de non utilisation de l'appareil. Bien entendu, pour pouvoir imprimer une empreinte dans ce cas, le couvercle 2b doit être complètement ouvert. Il n'est d'ailleurs pas indispensable qu'il soit relié à la partie inférieure 2a du boîtier par une articulation.

D'autre part, au lieu d'être fixée seulement le long de deux côtés opposés, la membrane 4 pourrait être fixée au couvercle 2b ou à la partie inférieure 2a le long de ses quatre côtés.

En outre, bien que dans le mode de réalisation décrit plus haut, la tirette 7 soit prévue pour supporter à la fois le rouleau encreur 6 et la feuille de papier 8, cette dernière pourrait être supportée directement par le fond 2c du boîtier 2, éventuellement dans un évidement peu profond de celui-ci. Dans ce cas, la tirette 7 peut être par exemple réalisée sous la forme d'un cadre présentant une large ouverture centrale afin que la membrane 4, lorsqu'elle est pressée, puisse venir en contact avec la feuille de papier placée sur le fond du boîtier. Cette dernière forme de réalisation offre l'avantage que la tirette 7 n'a pas besoin d'être réalisée avec une grande rigidité pour pouvoir supporter la pression d'impression et, du fait qu'elle présente une large ouverture centrale, on réalise ainsi une économie de matière pour la fabrication de la tirette.

## Revendications

1. Appareil permettant de réaliser des empreintes par impression à l'encre, comprenant un boîtier (2), un support (7) ayant une surface d'appui pour supporter au moins une feuille de papier (8) destinée à recevoir une empreinte, une membrane (4) en une matière élastomère, qui est tendue dans le boîtier et possède une première face orientée vers ladite feuille de papier et une seconde face dont une grande partie au moins est exposée à l'extérieur du boîtier, et un rouleau encreur (6) qui est en contact avec la première face de la membrane et fixé à une tirette (7) montée mobile dans le boîtier pour déplacer le rouleau encreur le long de la première face de la membrane afin d'encrer celle-ci, ledit rouleau encreur comportant un noyau cylindrique dur (6a) pourvu d'une garniture extérieure (6b) imprégnée d'encre, caractérisé en ce que ladite garniture (6b) est constituée par un bandage cylindrique épais en une matière plastique spongieuse microporeuse contenant dans sa masse une encre à base de solvants lourds, non volatils, hygroscopiques, non plastifiants pour ladite matière plastique et capables d'exsuder lentement vers l'extérieur du bandage pour assurer l'encrage.

2. Appareil selon la revendication 1, caractérisé en ce que la tirette (7) comporte, à une de ses extrémités, deux flasques espacés (7b) qui sont d'un seul tenant avec la tirette et servent de paliers pour le rouleau encreur (6).

3. Appareil selon la revendication 2, caractérisé en ce qu'à chacune de ses extrémités le noyau (6a) du rouleau encreur (6) est pourvu d'un tourillon (9) qui est monté à rotation dans l'un desdits flasques (7b).

4. Appareil selon la revendication 3, caractérisé en ce que chaque flasque (7b) comporte une encoche (11) ayant un profil en partie circulaire, dont le rayon correspond au diamètre des tourillons (9) du rouleau encreur (6) et qui s'étend sur un arc d'un peu plus de 180°, chaque tourillon (9) étant introduit à force dans l'encoche (11) du flasque (7b) correspondant et maintenu dans celle-ci par encliquetage élastique.

5. Appareil selon l'une quelconque des revendications 2 à 4, caractérisé en ce que la tirette (7) est en contact glissant avec le fond (2c) du boîtier (2), passe à travers une fente (12) prévue dans l'une des parois latérales du boîtier (2), près du fond de celui-ci, et comporte une partie (7c) qui fait saillie à l'extérieur du boîtier et qui sert de poignée pour la manoeuvre de la tirette (7).

6. Appareil selon la revendication 5, caractérisé en ce que la membrane (4) est maintenue le long d'au moins deux côtés opposés à une distance prédéterminée du fond (2c) du boîtier (2), et en ce que les dimensions des flasques (7b) et le diamètre extérieur du bandage cylindrique (6b) sont tels que la partie périphérique dudit bandage qui est en contact avec la membrane (4) soit à une distance du fond (2c) du boîtier sensiblement plus grande que ladite distance prédéterminée, de telle sorte que la membrane soit fermement appliquée élastiquement contre ledit bandage.
